# EUROPEAN PATENT APPLICATION

(11) **EP 1 127 573 A1**
(43) Date of publication of application: **29.08.2001**
(21) Application number: 01301276.0
(22) Date of filing: 13.02.2001
(51) Int. Cl.: A61K 31/66, A61P 9/00, A61P 19/10

(54) **Compostitions and methods for treating osteoporosis**

(30) Priority: 15.02.2000 US 182713
(71) Applicant: Pfizer Products Inc., Groton, Connecticut 06340 (US)
(72) Inventor: Day, Wesley Warren, Pfizer Global Res. & Dev., Connecticut 06340 (US); Lee, Andrew George, Pfizer Global Res. & Dev., Connecticut 06340 (US); Thompson, David D., Pfizer Global Res. & Dev., Connecticut 06340 (US)
(74) Representative: Ruddock, Keith Stephen

(57) **Abstract**

This invention relates to methods, pharmaceutical compositions and kits useful in promoting bone formation and/or preventing bone loss and/or treating atherosclerosis. The compositions are comprised of a polyphosphonate as a first active component and a statin as a second active component and a pharmaceutically acceptable vehicle, carrier or diluent.

## Description

### FIELD OF THE INVENTION

This invention relates to pharmaceutical compositions comprising combinations of polyphosphonates and statins, and pharmaceutically acceptable salts thereof, kits comprising such combinations and methods of using such combinations to prevent bone loss and/or promote bone formation and/or treat atherosclerosis. The compositions and methods are useful for treating subjects suffering from osteoporosis, bone fracture or deficiency, primary or secondary hyperparathyroidism, periodontal disease, metastatic bone disease, osteolytic bone disease, or undergoing orthopedic or oral surgery.

### BACKGROUND OF THE INVENTION

A variety of disorders in humans and other mammals involve or are associated with abnormal bone resorption. Such disorders include, but are not limited to, osteoporosis, Paget's disease, periprosthetic bone loss or osteolysis, metastatic bone disease, hypercalcemia of malignancy, multiple myeloma, periodontal disease, and tooth loss. The most common of these disorders is osteoporosis, which in its most frequent manifestation occurs in postmenopausal women. Osteoporosis is a systemic skeletal disease characterized by a low bone mass and microarchitectural deterioration of bone tissue, with a consequent increase in bone fragility and susceptibility to fracture. Because osteoporosis, as well as other disorders associated with bone loss, are chronic conditions, it is believed that appropriate therapy will generally require chronic treatment.

Multinucleated cells called osteoclasts are responsible for causing bone loss through a process known as bone resorption. Polyphosphonates are selective inhibitors of osteoclastic bone resorption, making these compounds important therapeutic agents in the treatment or prevention of a variety of generalized or localized bone disorders caused by or associated with abnormal bone resorption. See H. Fleisch, Bisphosphonates In Bone Disease, From The Laboratory To The Patient, 2nd Edition, Parthenon Publishing (1995).

At present, a great amount of preclinical and clinical data exists for the polyphosphonate compound alendronate. Evidence suggests that other polyphosphonates such as risedronate, tiludronate, ibandronate and zolendronate, have many properties in common with alendronate, including high potency as inhibitors of osteoclastic bone resorption. An older polyphosphonate compound, etidronate, also inhibits bone resorption. However, unlike the more potent polyphosphonates, etidronate impairs mineralization at doses used clinically, and may give rise to osteomalacia, a condition resulting in an undesirable decrease in bone mineralization (Boyce, B. F., Fogelman, I., Ralston, S. et al. Lancet 1984;8381:821-824, and Gibbs, C. J., Aaron, J. E.; Peacock, M., Br. Med. J., 1986;292:1227-1229.

Statins exhibit a bone-forming effect in addition to a cholesterol-lowering effect. Statins inhibit the enzyme HMG-CoA reductase that catalyzes the conversion of 3-hydroxy-3-methylglutaryl-coenzyme A (HMG-CoA) to mevalonate in an early and rate-limiting step in the cholesterol biosynthetic pathway. It is believed that this effect is responsible for statins being considered as potent lipid lowering agents. The bone-forming effect of statins may be due to their ability to increase bone formation rate possibly through the stimulation of growth factors such as bone morphogenic protein-2 (BMP-2) (Mundy, G., et al., Science, 1999;286:1946-1949).

Statins include such compounds as simvastatin, disclosed in U.S. 4,444,784; pravastatin, disclosed in U.S. 4,346,227; cerivastatin, disclosed in U.S. 5,502,199; mevastatin, disclosed in U.S. 3,983,140; velostatin, disclosed in U.S. 4,448,784 and U.S. 4,450,171; fluvastatin, disclosed in U.S. 4,739,073; compactin, disclosed in U.S. 4,804,770; lovastatin, disclosed in U.S. 4,231,938; dalvastatin, disclosed in European Patent Application Publication No. 738510 A2; fluindostatin, disclosed in European Patent Application Publication No. 363934 A1; atorvastatin, disclosed in U.S. Patent No. 4,681,893; atorvastatin hemicalcium salt, disclosed in U.S. Patent No. 5,273,995; dihydrocompactin, disclosed in U.S. 4,450,171; ZD-4522, disclosed in U.S. Patent No. 5,260,440; bervastatin, disclosed in U.S. Patent No. 5,082,859; and NK-104, disclosed in U.S. Patent No. 5,102,888.

Bone is a tissue that is subject to turnover. Bone homeostasis is balanced by the osteoblasts that produce new bone and the osteoclasts that destroy bone. The activities of these cells are regulated by a large number of cytokines and growth factors, many of-which have now been identified and cloned. Mundy has described the current knowledge related to these factors (Mundy, G. R. Clin Orthop 1996;324:24-28; Mundy, G. R. J Bone Miner Res 1993;8:S505-10).

Growth factors that stimulate bone formation have been identified. Among these factors are transforming growth factor, the heparin-binding growth factors (acidic and basic fibroblast growth factor), the insulin-like growth factors (insulin-like growth factor I and insulin-like growth factor II), and a recently described family of proteins called bone morphogenetic proteins (BMPs). All of these growth factors have effects on other types of cells, as well as on bone cells. The BMPs are novel factors in the extended transforming growth factor *β* superfamily. The BMPs were identified by Wozney J. et al. Science 1988;242: 1528-34, following earlier descriptions characterizing the biological activity in extracts of demineralized bone (Urist M. Science 1965;150: 893-99). Recombinant BMP2 and BMP4 can induce new bone formation when they are injected locally into the subcutaneous tissues of rats (Wozney J. Molec Reprod Dev 1992;32:160-67). These factors are expressed by normal osteoblasts as they differentiate, and have been shown to stimulate osteoblast differentiation and bone nodule formation in vitro as well as bone formation in vivo (Harris S. et al. J. Bone Miner Res 1994;9:855-63).

As osteoblasts differentiate from precursors to mature bone-forming cells, they express and secrete a number of enzymes and structural proteins of the bone matrix, including Type-1 collagen, osteocalcin, osteopontin and alkaline phosphatase (Stein G. et al. Curr Opin Cell Biol 1990;2:1018-27; Harris S. et al. (1994), supra). They also synthesize a number of growth regulatory peptides which are stored in the bone matrix, and are presumably responsible for normal bone formation. These growth regulatory peptides include the BMPs (Harris S. et al. (1994), supra). In studies of primary cultures of fetal rat calvarial osteoblasts, BMPs 1, 2, 3, 4,and 6 are expressed by cultured cells prior to the formation of mineralized bone nodules (Harris S. et al. (1994), supra). Like alkaline phosphatase, osteocalcin and osteopontin, the BMPs are expressed by cultured osteoblasts as they proliferate and differentiate.

### SUMMARY OF THE INVENTION

This invention relates to pharmaceutical compositions useful for promoting bone formation and/or preventing bone loss and/or treating atherosclerosis. The compositions are comprised of a bone resorption inhibiting polyphosphonate and a statin and, optionally, a pharmaceutically acceptable carrier, vehicle or diluent. The compositions exert an effect which is additive or greater than the sum of the individual effects of the bone resorption inhibiting polyphosphonates and statins when administered separately.

A second aspect of the invention relates to methods of promoting bone formation and/or preventing bone loss and/or treating atherosclerosis. The methods comprise the administration of an effective amount of the pharmaceutical compositions comprising a bone resorption inhibiting polyphosphonate and a statin as described herein or co-administration of a polyphosphonate and a statin.

As a third aspect, the present invention provides for kits for use by a consumer to promote bone formation and/or prevent bone loss and/or treat atherosclerosis. The kits comprise: a) a pharmaceutical composition comprising a bone resorption inhibiting polyphosphonate and a pharmaceutically acceptable carrier, vehicle or diluent; b) a pharmaceutical composition comprising a statin and a pharmaceutically acceptable carrier, vehicle or diluent; and, optionally, c) instructions describing a method of using the pharmaceutical compositions for promoting bone formation and/or preventing bone loss and/or treating atherosclerosis. The instructions may also indicate that the kit is for promoting bone formation and/or preventing bone loss and/or treating atherosclerosis or another specific condition related to these effects. The bone resorption inhibiting polyphosphonate and the statin contained in the kit may be optionally combined in the same pharmaceutical composition.

As a fourth aspect, the present invention provides for the use of bone resorption inhibiting polyphosphonate and statins for the manufacture of a medicament to promote bone formation and/or prevent bone loss and/or treat atherosclerosis.

A fifth aspect of the invention is that the compositions and methods of the invention can further comprise a histamine H2 receptor blocker (i.e. antagonist) and/or a proton pump blocker.

### DETAILED DESCRIPTION OF THE INVENTION

The present invention relates to compositions and methods for promoting bone formation and/or preventing bone loss and/or treating atherosclerosis. Unless otherwise specified, the following terms have the meanings as defined below:

As used herein, "limit", "treat" and "treatment" are interchangeable terms as are "limiting" and "treating" and, as used herein, include preventative (e.g., prophylactic) and palliative treatment or the act of providing preventative or palliative treatment. The terms include a postponement of development of bone deficit symptoms and/or a reduction in the severity of such symptoms that will or are expected to develop. The terms further include ameliorating existing bone or cartilage deficit symptoms, preventing additional symptoms, ameliorating or preventing the underlying metabolic causes of symptoms, preventing or reversing bone resorption and/or encouraging bone growth. By "bone deficit" is meant an imbalance in the ratio of bone formation to bone resorption, such that, if unmodified, the subject will exhibit less bone than desirable, or the subject's bones will be less intact than desired. Bone deficit may also result from fracture, from surgical intervention or from dental or periodontal disease. By "cartilage defect" is meant damaged cartilage, less cartilage than desired, or cartilage that is less intact than desired. The terms, "limit", "treat" and "treatment and "limiting" and "treating" further include the lowering of existing blood cholesterol levels and the prevention of the elevation of blood cholesterol levels and the symptoms and conditions caused or related to the blood cholesterol levels such as atherosclerosis and hyperlipidemia, or increased cardiac risk and the inhibition of calcification of atherosclerotic plaques or the stabilization of atherosclerotic plaques.

Representative uses of the compositions and methods of the present invention include: repair of bone defects and deficiencies, such as those occurring in closed, open and nonunion fractures; prophylactic use in closed and open fracture reduction; promotion of bone healing in plastic surgery; stimulation of bone ingrowth into non-cemented prosthetic joints and dental implants; elevation of peak bone mass in perimenopausal women, treatment of growth deficiencies; treatment of periodontal disease and defects, and other tooth repair processes; increase in bone formation during distraction osteogenesis; and treatment of other skeletal disorders, such as age-related osteoporosis, post-menopausal osteoporosis, glucocorticoid-induced osteoporosis or disuse osteoporosis and arthritis, or any condition that benefits from stimulation of bone formation. The compositions and methods of the present invention can also be useful in repair of congenital, trauma-induced or surgical resection of bone (for instance, for cancer treatment), and in cosmetic surgery. Further, the compositions and methods of the present invention can be used for treating cartilage defects or disorders, and are useful in wound healing or tissue repair. Additionally, the compositions and methods of the present invention can be used to treat atherosclerosis.

Bone or cartilage deficit or defect and atherosclerosis can be treated in vertebrate subjects by administering the compositions of the invention. The compositions of the invention may be administered systemically or locally. For systemic use, the compounds herein are formulated for parenteral (e.g., intravenous, subcutaneous, intramuscular, intraperitoneal, intranasal or transdermal) or enteral (e.g., oral or rectal) delivery according to conventional methods. Intravenous administration can be by a series of injections or by continuous infusion over an extended period. Administration by injection or other routes of discretely spaced administration can be performed at intervals ranging from weekly to once to three times daily or more. Alternatively, the compositions disclosed herein may be administered in a cyclical manner (administration of disclosed composition, followed by no administration, followed by administration of disclosed compositions, and the like). Treatment will continue until the desired outcome is achieved.

A "subject" is an animal including a human that is in need of treatment with the compositions, methods and kits of the present invention. The term "subject" or "subjects" is intended to refer to both the male and female gender unless one gender is specifically indicated.

The term "post-menopausal women" is defined to include not only women of advanced age who have passed through menopause, but also women who have been hysterectomized or for some other reason have suppressed estrogen production, such as those who have undergone long-term administration of corticosteroids, suffer from Cushions' syndrome or have gonadal dysgenesis.

"Co-administration" of a combination of a statin and a polyphosphonate means that these components can be administered together as a composition or as part of the same, unitary dosage form. "Co-administration" also includes administering a statin and a polyphosphonate separately but as part of the same therapeutic treatment program or regimen. The components need not necessarily be administered at essentially the same time, although they can if so desired. Thus "co-administration" includes, for example, administering a statin and a polyphosphonate as separate dosages or dosage forms, but at the same time. "Co-administration" also includes separate administration at different times and in any order. For example, where appropriate a patient may take one or more component(s) of the treatment in the morning and the one or more of the other component(s) at night.

A statin and a bone resorption inhibiting polyphosphonate when co-administered either as part of the same pharmaceutical composition or as separate pharmaceutical compositions is/are effective in promoting bone formation and/or preventing bone loss and/or treating atherosclerosis. By producing these effects, the compositions and methods of the invention are suitable for treating a variety of conditions. These conditions include osteoporosis, including age-related osteoporosis and osteoporosis associated with post-menopausal hormone status. Other conditions characterized by the need for bone growth include primary and secondary hyperparathyroidism, disuse osteoporosis, diabetes-related osteoporosis, and glucocorticoid-related osteoporosis. The results of the methods in enhancing bone formation make the compositions and methods useful for bone repair and bone deficit conditions. Such conditions include bone fracture and facial reconstruction surgery and bone segmental defects, periodontal disease, metastatic bone disease, osteolytic bone disease and conditions where connective tissue repair is beneficial, such as healing or regeneration of cartilage defects or injury. Additionally the compositions and methods are useful for treating atherosclerosis and hyperlipidemia and for preventing calcification of atherosclerotic plaques or stabilizing such plaques.

By "bone resorption inhibiting polyphosphonate" as used herein is meant a polyphosphonate such as the type disclosed in U.S. Patent No. 3,683,080 or formula I below. Preferred polyphosphonates are geminal diphosphonates (also referred to as bisphosphonates). The polyphosphonates may be administered in the form of the acid, or of a soluble alkali metal salt or alkaline earth metal salt. Polyphosphonates of the present invention include those of chemical formula I: 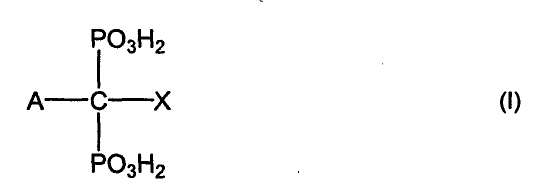 wherein

A and X are independently selected from the group consisting of H, OH, halogen, NH₂, SH, phenyl, C₁-C₃₀ alkyl, C₁-C₃₀ substituted alkyl, C₁-C₁₀ alkyl or dialkyl substituted NH₂, C₁-C₁₀ alkoxy, C₁-C₁₀ alkyl or phenyl substituted thio, C₁ - C₁₀ alkyl substituted phenyl, pyridyl, furanyl, pyrrolidinyl, imidazonyl, and benzyl.

In the foregoing chemical formula, the alkyl groups can be straight, branched, or cyclic. The C₁-C₃₀ substituted alkyl can include a wide variety of substituents, nonlimiting examples of which include those selected from the group consisting of phenyl, pyridyl, furanyl, pyrrolidinyl, imidazonyl, NH₂, and C₁-C₁₀ alkyl or dialkyl substituted NH₂, OH, SH, and C₁-C₁₀ alkoxy.

In the foregoing chemical formula, A can include X and X can include A such that the two moieties can form part of the same cyclic structure.

The foregoing chemical formula is also intended to encompass complex carbocyclic, aromatic and hetero atom structures for the A and/or X substituents, nonlimiting examples of which include naphthyl, quinolyl, isoquinolyl, adamantly, and chlorophenylthio.

Preferred structures are those in which A is selected from the group consisting of H, OH, and halogen, and X is selected from the group consisting of C₁-C₃₀ alkyl, C₁-C₃₀ substituted alkyl, halogen, and C₁-C₁₀ alkyl or phenyl substituted thio.

More preferred structures are those in which A is selected from the group consisting of H, OH, and Cl, and X is selected from the group consisting of C₁-C₃₀ alkyl, C₁-C₃₀ substituted alkyl, Cl, and chlorophenylthio.

Most preferred is when A is OH and X is a 3-aminopropyl moiety, so that the resulting compound is a 4-amino-1-hydroxybutylidene-1,1-bisphosphonate, i.e. alendronate.

Pharmaceutically acceptable salts and derivatives of the polyphosphonates are also useful herein. Nonlimiting examples of salts include those selected from the group consisting alkali metal, alkaline metal, ammonium, and mono-, di, tri-, or tetra-C₁-C₃₀-alkyl-substituted ammonium.

Preferred salts are those selected from the group consisting of sodium, potassium, calcium, magnesium, and ammonium salts. Nonlimiting examples of derivatives include those selected from the group consisting of esters, hydrates, and amides.

The terms "polyphosphonate", "bisphosphonate" and "bisphosphonates", as used herein in referring to the therapeutic agents of the present invention are meant to also encompass diphosphonates, biphosphonic acids, and diphosphonic acids, as well as salts and derivatives of these materials and are examples of bone resorption inhibiting polyphosphonates. The use of a specific nomenclature in referring to the bisphosphonate or bisphosphonates is not meant to limit the scope of the present invention, unless specifically indicated. Because of the mixed nomenclature currently in use by those of ordinary skill in the art, reference to a specific weight or percentage of a polyphosphonate compound in the present invention is on an acid active weight basis, unless indicated otherwise herein.

Nonlimiting examples of polyphosphonates useful herein include the following:
a) alendronic acid, 4-amino-1-hydroxybutylidene-1,1-bisphosphonic acid;
b) alendronate (also known as alendronate sodium or monosodium trihydrate), 4-amino-1-hydroxybutylidene-1,1-bisphosphonic acid monosodium trihydrate;
c) alendronic acid and alendronate are described in U.S. Pat. No. 4,922,007, to Kieczykowski et al., issued May 1, 1990, and U.S. Pat. No. 5,019,651, to Kieczykowski, issued May 28, 1991;
d) cycloheptylaminomethylene-1,1-bisphosphonic add, YM 175, Yamanouchi (cimadronate), as described in U.S. Pat. No. 4,970,335, to Isomura et al., issued Nov. 13, 1990;
e) 1,1-dichloromethylene-1,1-diphosphonic acid (clodronic acid), and the disodium salt (clodronate, Procter and Gamble), are described in Belgium Patent 672,205 (1966) and J. Org. Chem., 1967;32:4111;
f) 1-hydroxy-3-(1-pyrrolidinyl)-propylidene-1,1-bisphosphonic acid (EB-1053);
g) 1-hydroxyethane-1,1-diphosphonic acid (etidronic acid);
h) 1-hydroxy-3-(N-methyl-N-pentylamino)propylidene-1,1-bisphosphonic acid, also known as BM-210955, Boehringer-Mannheim (ibandronate), is described in U.S. Pat. No. 4,927,814, issued May 22, 1990;
i) 6-amino-1-hydroxyhexylidene-1,1-bisphosphonic acid (neridronate);
j) 3-(dimethylamino)-1-hydroxypropylidene-1,1-bisphosphonic acid (olpadronate);
k) 3-amino-1-hydroxypropylidene-1,1-bisphosphonic acid (pamidronate);
I) [2-(2-pyridinyl)ethylidene]- 1,1 -bisphosphonic acid (piridronate) as described in U.S. Pat. No. 4,761,406;
m) 1-hydroxy-2-(3-pyridinyl)-ethylidene-1,1-bisphosphonic acid (risedronate);
n) (4-chlorophenyl)thiomethane-1,1-disphosphonic acid (tiludronate) as described in U.S. Pat. No. 4,876,248, to Breliere et al., Oct. 24, 1989; and
o) 1-hydroxy-2-(1H-imidazol-1-yl)ethylidene-1,1-bisphosphonic acid (zolendronate).

Preferred are polyphosphonates selected from the group consisting of alendronate, cimadronate, clodronate, tiludronate, etidronate, ibandronate, risedronate, piridronate, pamidronate, zolendronate, pharmaceutically acceptable salts thereof, and mixtures thereof.

More preferred is alendronate, pharmaceutically acceptable salts thereof, and mixtures thereof with alendronate monosodium trihydrate being the most preferred.

The precise dosage of the polyphosphonate will vary with the dosing schedule, the oral potency of the particular polyphosphonate chosen, the age, size, sex and condition of the mammal, the nature and severity of the disorder to be treated, and other relevant medical and physical factors. Thus, a precise pharmaceutically effective amount cannot be specified in advance and can be readily determined by the caregiver or clinician.

Generally, an appropriate amount of polyphosphonate is chosen to obtain a bone resorption inhibiting effect, i.e. a bone resorption inhibiting amount of the polyphosphonate is administered. For humans, an effective oral dose of polyphosphonate is typically from about 1.5 to about 6000 µg/kg body weight and preferably about 10 to about 2000 µg/kg of body weight.

For human oral compositions comprising alendronate, a pharmaceutically acceptable salt thereof, or a pharmaceutically acceptable derivative thereof, a unit dosage typically comprises from about 8.75 mg to about 140 mg of the alendronate compound, on an alendronic acid active weight basis.

For once-weekly dosing, an oral unit dosage comprises from about 17.5 mg to about 70 mg of the alendronate compound, on an alendronic acid active weight basis. Examples of weekly oral dosages include a unit dosage which is useful for osteoporosis prevention comprising about 35 mg of the alendronate compound, and a unit dosage which is useful for treating osteoporosis comprising about 70 mg of the alendronate compound.

For twice-weekly dosing, an oral unit dosage comprises from about 8.75 mg to about 35 mg of the alendronate compound, on an alendronic acid active weight basis. Examples of twice-weekly oral dosages include a unit dosage which is useful for osteoporosis prevention comprising about 17.5 mg of the alendronate compound, and a unit dosage which is useful for osteoporosis treatment, comprising about 35 mg of the alendronate compound.

For biweekly or twice-monthly dosing, an oral unit dosage comprises from about 35 mg to about 140 mg of the alendronate compound, on an alendronic acid active weight basis. Examples of biweekly or twice-monthly oral dosages include a unit dosage which is useful for useful for osteoporosis prevention comprising about 70 mg of the alendronate compound, and a unit dosage which is useful for osteoporosis treatment, comprising. about 140 mg of the alendronate compound.

In further embodiments, the methods and compositions of the present invention can also comprise a histamine H₂ receptor blocker (i.e. antagonist) and/or a proton pump inhibitor. Histamine H₂ receptor blockers and proton pump inhibitors are well known therapeutic agents for increasing gastric pH. See L. J. Hixson, et al., Current Trends in the Pharmacotherapy for Peptic Ulcer Disease, Arch. Intern. Med., 1992;152:726-732. It is found in the present invention that the sequential oral administration of a histamine H₂ receptor blocker and/or a proton pump inhibitor, followed by a polyphosphonate can help to further minimize adverse gastrointestinal effects. In these embodiments, the histamine H₂ receptor blocker and/or proton pump inhibitor is administered from about 30 minutes to about 24 hours prior to the administration of the polyphosphonate. In more preferred embodiments, the histamine H₂ receptor blocker and/or proton pump inhibitor is administered from about 30 minutes to about 12 hours prior to the administration of the polyphosphonate.

The dosage of the histamine H₂ receptor blocker and/or proton pump inhibitor will depend upon the particular compound selected and factors associated with the mammal to be treated, i.e. size, health, etc.

Nonlimiting examples of histamine H₂ receptor blockers and/or proton pump inhibitors include those selected from the group consisting of cimetidine, famotidine, nizatidine, ranitidine, omeprazole, and lansoprazole.

The other active component of the combinations of this invention is a statin. The term "statin", where used in the description and the appendant claims, is synonymous with the terms "3-hydroxy-3-methylglutaryl-Coenzyme A reductase inhibitor" and "HMG-CoA reductase inhibitor." These three terms are used interchangeably throughout the description and appendant claims. As the synonyms suggest, statins are inhibitors of 3-hydroxy-3-methylglutaryl-Coenzyme A reductase and as such are effective in lowering the level of blood plasma cholesterol and promoting bone formation. Statins and pharmaceutically acceptable salts thereof are particularly useful in preventing bone loss and/or promoting bone formation and in lowering low density lipoprotein cholesterol (LDL-C) levels in mammals and particularly in humans.

The statins suitable for use herein include, but are not limited to, simvastatin, pravastatin, cerivastatin, mevastatin, fluindostatin, velostatin, fluvastatin, dalvastatin, dihydrocompactin, compactin, lovastatin, atorvastatin, bervastatin, NK-104 and ZD-4522 and pharmaceutically acceptable salts thereof.

The statins disclosed herein are prepared by methods well known to those skilled in the art. Specifically, simvastatin may be prepared according to the method disclosed in U.S. 4,444,784. Pravastatin may be prepared according to the method disclosed in U.S. 4,346,227. Cerivastatin may be prepared according to the method disclosed in U.S. 5,502,199. Cerivastatin may alternatively be prepared according to the method disclosed in European Patent Application Publication No. EP617019. Mevastatin may be prepared according to the method disclosed in U.S. 3,983,140. Velostatin may be prepared according to the methods disclosed in U.S. 4,448,784 and U.S. 4,450,171. Fluvastatin may be prepared according to the method disclosed in U.S. 4,739,073. Compactin may be prepared according to the method disclosed in U.S. 4,804,770. Lovastatin may be prepared according to the method disclosed in U.S. 4,231,938. Dalvastatin may be prepared according to the method disclosed in European Patent Application Publication No. EP738510. Fluvastatin may be prepared according to the method disclosed in European Patent Application Publication No. EP363934. Dihydrocompactin may be prepared according to the method disclosed in U.S. 4,450,171. Atorvastatin may be prepared according to the methods disclosed in U.S. 4,681,893 and U.S. 5,273,995. Bervastatin, as shown in formula II below, may be prepared according to the methods disclosed in U.S. Patent No. 5,082,859. NK-104, as shown in formula III below, may be prepared by the methods disclosed in U.S. Patent No. 5,102,888. ZD-4522, shown in formula IV below, may be prepared by the methods disclosed in U.S. Patent No. 5,260,440.

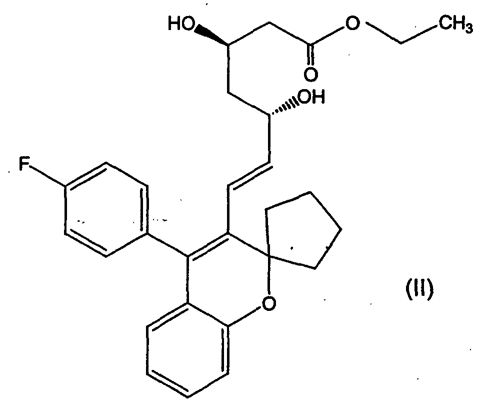

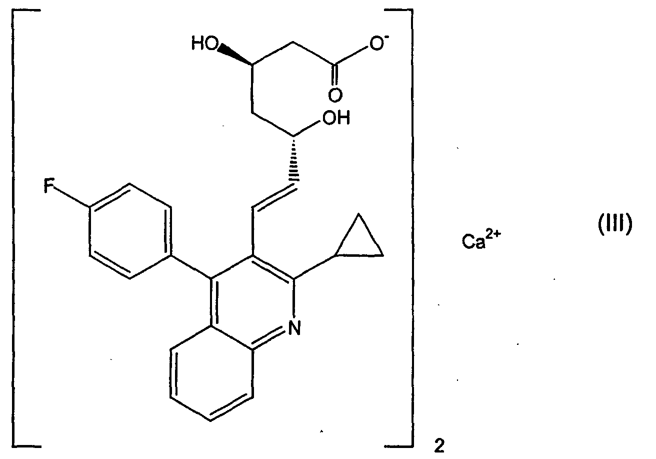

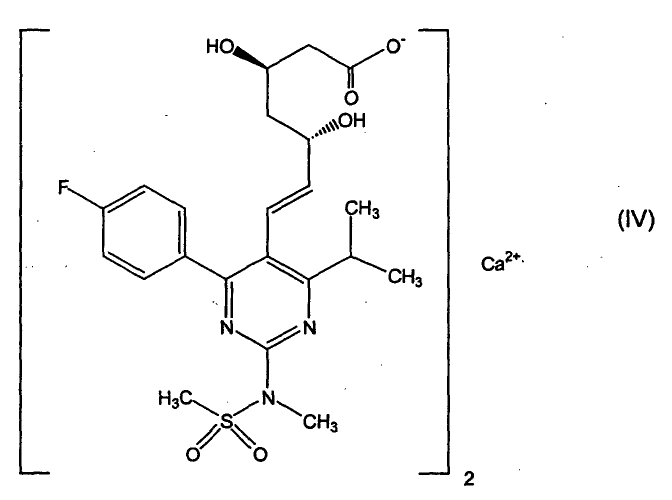

It will be recognized that certain of the above bone resorption inhibiting polyphosphonates and statins contain either a free carboxylic acid or a free amine group as part of the chemical structure. Further, certain polyphosphonates and statins within the scope of this invention contain lactone moieties, which exist in equilibrium with the free carboxylic acid form. These lactones can be maintained as carboxylates by preparing pharmaceutically acceptable salts of the lactone. Thus, this invention includes pharmaceutically acceptable salts of those carboxylic acids or amine groups. The expression "pharmaceutically acceptable salts" includes both pharmaceutically acceptable acid addition salts and pharmaceutically acceptable cationic salts. "Pharmaceutically acceptable salts" further include mutual salts formed between statins and polyphosphonates. The expression "pharmaceutically-acceptable cationic salts" is intended to define but is not limited to such salts as the alkali metal salts, (e.g. sodium and potassium), alkaline earth metal salts (e.g. calcium and magnesium), aluminum salts, ammonium salts, and salts with organic amines such as benzathine (N,N'-dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol) and procaine. The expression "pharmaceutically-acceptable acid addition salts" is intended to define but is not limited to such salts as the hydrochloride, hydrobromide, sulfate, hydrogen sulfate, phosphate, hydrogen phosphate, dihydrogenphosphate, acetate, succinate, citrate, methanesulfonate (mesylate) and p-toluenesulfonate (tosylate) salts.

The pharmaceutically-acceptable cationic salts of statins and polyphosphonates containing free carboxylic acids may be readily prepared by reacting the free acid form of the statin and/or polyphosphonate with an appropriate base, usually one equivalent, in a co-solvent. Typical bases are sodium hydroxide, sodium methoxide, sodium ethoxide, sodium hydride, potassium methoxide, magnesium hydroxide, calcium hydroxide, benzathine, choline, diethanolamine, piperazine and tromethamine. The salt is isolated by concentration to dryness or by addition of a non-solvent. In many cases, salts are preferably prepared by mixing a solution of the acid with a solution of a different salt of the cation (sodium or potassium ethylhexanoate, magnesium oleate), employing a solvent (e.g., ethyl acetate) from which the desired cationic salt precipitates, or can be otherwise isolated by concentration and/or addition of a non-solvent. In this manner, mutual salts of the statins may also be prepared with polyphosphonates.

The pharmaceutically acceptable acid addition salts of statins and polyphosphonates containing free amine groups may be readily prepared by reacting the free base form of the statin and/or polyphosphonate with the appropriate acid. When the salt is of a monobasic acid (e.g., the hydrochloride, the hydrobromide, the p-toluenesulfonate, the acetate), the hydrogen form of a dibasic acid (e.g., the hydrogen sulfate, the succinate) or the dihydrogen form of a tribasic acid (e.g., the dihydrogen phosphate, the citrate), at least one molar equivalent and usually a molar excess of the acid is employed. However when such salts as the sulfate, the hemisuccinate, the hydrogen phosphate or the phosphate are desired, the appropriate and exact chemical equivalents of acid will generally be used. The free base and the acid are usually combined in a co-solvent from which the desired salt precipitates, or can be otherwise isolated by concentration and/or addition of a non-solvent. Mutual salts of statins and polyphosphonates can be similarly prepared in this manner: For example, the mutual salt of atorvastatin and alendronic acid.

One of ordinary skill in the art will recognize that certain bone resorption inhibiting polyphosphonates and statins of this invention will contain one or more atoms which may be in a particular stereochemical, tautomeric, or geometric configuration, giving rise to stereoisomers, tautomers and configurational isomers. All such isomers and mixtures thereof are included in this invention. Hydrates and solvates of the compounds of this invention are also included.

The subject invention also includes isotopically-labeled bone resorption inhibiting polyphosphonates and statins, which are structurally identical to those disclosed above, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, sulfur, fluorine and chlorine, such as ²H, ³H, ¹³C, ¹⁴C, ¹⁵N, ¹⁸O, ¹⁷O, ³¹P, ³²P, ³⁵S, ¹⁸F and ³⁶Cl, respectively. Compounds of the present invention, derivatives thereof, and pharmaceutically acceptable salts of said compounds and of said derivatives which contain the aforementioned isotopes and/or other isotopes of other atoms are within the scope of this invention. Certain isotopically-labeled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, may afford certain therapeutic advantages resulting from greater metabolic stability, for example increased *in vivo* half-life or reduced dosage requirements and, hence, may be preferred in some circumstances. Isotopically labeled compounds of this invention and derivatives thereof can generally be prepared by carrying out known or referenced procedures and by substituting a readily available isotopically labeled reagent for a non-isotopically labeled reagent.

Those of ordinary skill in the art will recognize that physiologically active compounds which have accessible hydroxy groups are frequently administered in the form of pharmaceutically acceptable esters. The compounds of this invention can be effectively administered as an ester, formed on the hydroxy groups, just as one skilled in pharmaceutical chemistry would expect. It is possible, as has long been known in pharmaceutical chemistry, to adjust the rate or duration of action of the compound by appropriate choices of ester groups.

Certain ester groups are preferred as constituents of the compounds of this invention. The statins and/or compounds of formula I, II, III or IV may contain ester groups at various positions as defined herein above, where these groups are represented as -COOR⁹, R⁹ is C₁ -C₁₄ alkyl, C₁ -C₃ chloroalkyl, C₁ -C₃ fluoroalkyl, C₅ -C₇ cycloalkyl, phenyl, or phenyl mono- or disubstituted with C₁ -C₄ alkyl, C₁ -C₄ alkoxy, hydroxy, nitro, chloro, fluoro or tri(chloro or fluoro)methyl.

As used herein, the term "effective amount" means an amount of compound of the compositions, kits and methods of the present invention that is capable of treating the symptoms of the described conditions. The specific dose of a compound administered according to this invention will, of course, be determined by the particular circumstances surrounding the case including, for example, the compound administered, the route of administration, the state of being of the patient, and the severity of the condition being treated.

The dose of a compound of this invention to be administered to a subject is rather widely variable and subject to the judgement of the attending physician. It should be noted that it may be necessary to adjust the dose of a compound when it is administered in the form of a salt, such as a laureate, the salt forming moiety of which has an appreciable molecular weight.

The following dosage amounts and other dosage amounts set forth elsewhere in this description and in the appendant claims are for an average human subject having a weight of about 65 kg to about 70 kg. The skilled practitioner will readily be able to determine the dosage amount required for a subject whose weight falls outside the 65 kg to 70 kg range, based upon the medical history of the subject and the presence of diseases, e.g., diabetes, in the subject. Calculation of the dosage amount for other forms of the free base form such as salts or hydrates is easily accomplished by performing a simple ratio relative to the molecular weights of the species involved.

In general, in accordance with this invention, representative statins are administered in the following daily dosage amounts:
simvastatin, generally about 2.5 mg to about 160 mg and preferably about 10 mg to about 40 mg;
pravastatin, generally about 2.5 mg to about 160 mg and preferably about 10 mg to about 40 mg;
cerivastatin, generally about 25µg to about 5 mg and preferably about 1 mg to about 3.2 mg;
fluvastatin, generally about 2.5 mg to about 160 mg and preferably about 20 mg to about 80 mg;
lovastatin, generally about 2.5 mg to about 160 mg and preferably about 10 mg to about 80 mg; and
atorvastatin, generally about 2.5 mg to about 160 mg and preferably about 10 mg to about 80 mg.

In general, the pharmaceutical compositions will include a bone resorption inhibiting polyphosphonate as a first active ingredient and a statin as a second active ingredient in combination with a pharmaceutically acceptable vehicle, such as saline, buffered saline, 5% dextrose in water, borate-buffered saline containing trace metals or the like. Formulations may further include one or more excipients, preservatives, solubilizers, buffering agents, lubricants, fillers, stabilizers, etc. Methods of formulation are well known in the art and are disclosed, for example, in Remington's Pharmaceutical Sciences, Mack Publishing Company, Easton, Pa., 19th Edition (1995). Pharmaceutical compositions for use within the present invention can be in the form of sterile, non-pyrogenic liquid solutions or suspensions, coated capsules, suppositories, lyophilized powders, transdermal patches or other forms known in the art. The oral compositions may also include an H₂ histamine receptor blocker and/or a proton pump inhibitor. Local administration may be by injection at the site of injury or defect, or by insertion or attachment of a solid carrier at the site, or by direct, topical application of a viscous liquid, or the like. For local administration, the delivery vehicle preferably provides a matrix for the growing bone or cartilage, and more preferably is a vehicle that can be absorbed by the subject without adverse effects.

The active ingredient compounds are known to be absorbed from the alimentary tract, and so it is usually preferred to administer a compound orally for reasons of convenience. However, the compounds may equally effectively be administered percutaneously, locally at the site of injury or as suppositories for absorption by the rectum or vagina, if desired in a given instance. All of the usual types of compositions may be used, including tablets, chewable tablets, capsules, solutions, parenteral solutions, troches, suppositories and suspensions. Compositions are formulated to contain a daily dose, or a convenient fraction of daily dose, in a dosage unit, which may be a single tablet or capsule or convenient volume of a liquid.

Capsules are prepared by mixing the compound or compounds with a suitable diluent and filling the proper amount of the mixture in capsules. The usual diluents include inert powdered substances such as starch of many different kinds, powdered cellulose, especially crystalline and microcrystalline cellulose, sugars such as fructose, mannitol and sucrose, grain flours and similar edible powders.

Tablets are prepared by direct compression, by wet granulation, or by dry granulation. Their formulations usually incorporate diluents, binders, lubricants and disintegrators as well as the compound or compounds. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders.

A lubricant may be necessary in a tablet formulation to prevent the tablet and punches from sticking in the die. The lubricant is chosen from such slippery solids as talc, magnesium and calcium stearate, stearic acid and hydrogenated vegetable oils.

Tablet disintegrators are substances which swell when wetted to break up the tablet and release the compound or compounds. They include starches, clays, celluloses, algins and gums, more particularly, corn and potato starches, methylcellulose, agar, bentonite, wood cellulose, powdered natural sponge, cation-exchange resins, alginic acid, guar gum, citrus pulp and carboxymethylcellulose, for example, may be used as well as sodium lauryl sulfate.

Tablets are often coated with sugar as a flavor and sealant, or with film-forming protecting agents to modify the dissolution properties of the tablet. The compounds may also be formulated as chewable tablets, by using relatively large amounts of pleasant-tasting substances such as mannitol in the formulation, as is now well-established in the art.

When it is desired to administer a compound as a suppository, the typical bases may be used. Cocoa butter is a traditional suppository base, which may be modified by addition of waxes to raise its melting point slightly. Water-miscible suppository bases comprising, particularly, polyethylene glycols of various molecular weights are in wide use.

The effect of the compounds may be delayed or prolonged by proper formulation. For example, a slowly soluble pellet of the compound may be prepared and incorporated in a tablet or capsule. The technique may be improved by making pellets of several different dissolution rates and filling capsules with a mixture of the pellets. Tablets or capsules may be coated with a film which resists dissolution for a predictable period of time. Even the parenteral preparations may be made long-acting by dissolving or suspending the compound or compounds in oily or emulsified vehicles which allow dispersion slowly in the serum.

The combinations of this invention may be administered in a controlled release formulation such as a slow release or a fast release formulation. Such controlled release formulations of the combination of this invention may be prepared using methods well known to those skilled in the art. The method of administration will be determined by the attendant physician or other person skilled in the art after an evaluation of the subject's condition and requirements.

The term "prodrug" means compounds that are transformed in vivo to yield a compound of the present invention. The transformation may occur by various mechanisms, such as through hydrolysis in blood. A good discussion of the use of prodrugs is provided by T. Higuchi and W. Stella, "Pro-drugs as Novel Delivery Systems," Vol. 14 of the *A.C.S. Symposium Series,* and in *Bioreversible Carriers in Drug Design,* ed. Edward B. Roche, American Pharmaceutical Association and Pergamon Press, 1987. The term, "prodrug" also encompasses mutual prodrugs in which one or more statins are combined with one or more polyphosphonates in a single molecule that may then undergo transformation to yield the statins and polyphosphonates of the present invention.

For example, if a compound of the present invention contains a carboxylic acid functional group, a prodrug can comprise an ester formed by the replacement of the hydrogen atom of the acid group with a group such as (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl.

Similarly, if a compound of the present invention comprises an alcohol functional group, a prodrug can be formed by the replacement of the hydrogen atom of the alcohol group with a group such as (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, P(O)(OH)₂, -P(O)(O(C₁-C₆)alkyl)₂ or glycosyl (the radical resulting from the removal of a hydroxyl group of the hemiacetal form of a carbohydrate).

If a compound of the present invention comprises an amine functional group, a prodrug can be formed by the replacement of a hydrogen atom in the amine group with a group such as R^{X}-carbonyl, R^{X}O-carbonyl, NR^{X}R^{X}'-carbonyl where R^{X} and R^{X}' are each independently ((C₁-C₁₀)alkyl, (C₃-C₇)cycloalkyl, benzyl, or R^{X}-carbonyl is a natural α-aminoacyl or natural α-aminoacyl-natural α-aminoacyl, -C(OH)C(O)OY^{X} wherein (Y^{X} is H, (C₁-C₆)alkyl or benzyl), -C(OY^{X0}) Y^{X1} wherein Y^{X0} is (C₁-C₄) alkyl and Y^{X1} is ((C₁-C₆)alkyl, carboxy(C₁-C₆)alkyl, amino(C₁-C₄)alkyl or mono-N- or di-N,N-(C₁-C₆)alkylaminoalkyl, -C(Y^{X2}) Y^{X3} wherein Y^{X2} is H or methyl and Y^{X3} is mono-N- or di-N,N-(C₁-C₆)alkylamino, morpholino, piperidin-1-yl or pyrrolidin-1-yl.

Advantageously, the present invention also provides kits for use by a consumer for promoting bone formation and/or preventing bone loss and/or treating atherosclerosis. The kits comprise a) a pharmaceutical composition comprising a bone resorption inhibiting polyphosphonate and a pharmaceutically acceptable carrier, vehicle or diluent; b) a pharmaceutical composition comprising a statin and a pharmaceutically acceptable carrier, vehicle or diluent; and, optionally, c) instructions describing a method of using the pharmaceutical compositions for promoting bone formation and/or preventing bone loss and/or treating atherosclerosis. The polyphosphonate and the statin contained in the kit may be optionally combined in the same pharmaceutical composition.

A "kit" as used in the instant application includes a container for containing the pharmaceutical compositions such as a divided bottle or a divided foil packet. The container can be in any conventional shape or form as known in the art which is made of a pharmaceutically acceptable material, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. The container employed can depend on the exact dosage form involved, for example a conventional cardboard box would not generally be used to hold a liquid suspension. It is feasible that more than one container can be used together in a single package to market a single dosage form. For example, tablets may be contained in a bottle which is in turn contained within a box.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It maybe desirable to provide a written memory aid, where the written memory aid is of the type containing information and/or instructions for the physician, pharmacist or subject, e.g., in the form of numbers next to the tablets or capsules whereby the numbers correspond with the days of the regimen which the tablets or capsules so specified should be ingested or a card which contains the same type of information. Another example of such a memory aid is a calendar printed on the card e.g., as follows "First Week, Monday, Tuesday,"... etc.... "Second Week, Monday, Tuesday,..." etc. Other variations of memory aids will be readily apparent. A "daily dose" can be a single tablet or capsule or several tablets or capsules to be taken on a given day. Also a daily dose of one or more component(s) of the kit can consist of one tablet or capsule while a daily dose of another one or more component(s) of the kit can consist of several tablets or capsules.

Another specific embodiment of a kit is a dispenser designed to dispense the daily doses one at a time in the order of their intended use. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

Based on a reading of the present description and claims, certain modifications to the compositions and methods described herein will be apparent to one of ordinary skill in the art. The claims appended hereto are intended to encompass these modifications.

All references and patents cited herein are incorporated by reference.

### EXAMPLES

### Example 1: Effect of Bone Resorption Inhibiting Polyphosphonates and Statins in the Ovariectomized Rat Model: A Model of Post-Menopausal Osteoporosis.

In women, estrogen deficiency during the menopause results in increased bone turnover leading to bone loss. Ovariectomy in rats produces estrogen deficiency and increased bone turnover leading to trabecular bone loss similar to that observed in post-menopausal women (Kalu, D.N., Bone and Mineral 1991;15:175; Frost, H.M., Jee W.S.S., Bone and Mineral 1992;18:227; Wronski, T.J., Yen, C-F, Cells Materials 1991;(suppl. 1):69). The OVX rat is thus an appropriate model to evaluate compounds for the prevention and treatment of post-menopausal osteoporosis. The ability of bone resorption inhibiting polyphosphonates and statins alone and in combination to inhibit estrogen deficiency bone loss is assessed in OVX rats, since ovariectomy causes significant bone loss in the lumbar vertebrae, proximal tibia, and distal femoral metaphyses (Ke, H.Z., et al., Endocrin 1995;136:2435; Chen, H.K., et al., J Bone Miner Res 1995:10:1256).

Seventy-five day old female Sprague Dawley rats (weight range of 225 to 275 g) are obtained from Charles River Laboratories (Portage, Mich.). They are housed in groups of 3 and have ad libitum access to food (calcium content approximately 1%) and water. Room temperature is maintained at 22.2° ± 1.7°C. with a minimum relative humidity of 40%. The photoperiod in the room is 12 hours light and 12 hours dark. One week after arrival, the rats undergo bilateral ovariectomy under anesthesia (44 mg/kg Ketamine™ and 5 mg/kg Xylazine™ (Butler, Indianapolis, Ind.) administered intramuscularly). Treatment with vehicle or the test compositions is initiated either on the day of surgery following recovery from anesthesia or 35 days following the surgery. The rats are treated either with vehicle containing bone resorption inhibiting polyphosphonate or statin or bone resorption inhibiting polyphosphonate and statin or with vehicle only. Oral dosage is by gavage in 0.5 mL of pH-adjusted 1% carboxymethylcellulose (CMC). Body weight is determined at the time of surgery and weekly during the study, and the dosage is adjusted with changes in body weight. Vehicle-treated ovariectomized (OVX) rats and non-ovariectomized (intact) rats are evaluated in parallel with each experimental group to serve as negative and positive controls. The rats are treated daily for 35 days (6 rats per treatment group) and sacrificed by decapitation on the 36th day. The 35-day time period is sufficient to allow maximal reduction in bone density, measured as described below. At the time of sacrifice, the uteri are removed, dissected free of extraneous tissue, and the fluid contents are expelled before determination of wet weight in order to confirm estrogen deficiency associated with complete ovariectomy. Uterine weight is routinely reduced about 75% in response to ovariectomy. The uteri are then placed in 10% neutral buffered formalin to allow for subsequent histological analysis.

Calcein at 10 mg/kg is injected s.c. to all rats 12 and 2 days before necropsy as a fluorochrome bone marker to measure bone dynamic histomorphometric parameters. The effects of polyphosphonate, statin and combination polyphosphonate and statin on the following end points are determined: (a) serum osteocalcin, a biochemical marker of bone turnover, (b) bone mineral density of lumbar vertebrae and distal femoral metaphyses, (c) bone histomorphometry of fifth lumbar vertebral body and proximal tibial metaphyses.

For the measurement of the endpoints, serum osteocalcin concentration is determined by radioimmunoassay assays known in the art, and bone mineral content (BMC) and bone mineral density (BMD) are measured by standard procedures as described below:

The first to the sixth lumbar vertebrae from each rat are removed during necropsy. These were then scanned *ex vivo* using dual-energy X-ray absorptiometry. The scan images are analyzed, and bone area, BMC, and BMD of whole lumbar vertebrae (WLV), and LV1 through LV6 is determined.

Using dual-energy X-ray absorptiometry, the right femur of each rat is scanned *ex vivo.* Bone mineral density (BMD) of the distal femoral metaphyses (second 0.5 cm from the distal end of femur) and the proximal femur (the first 0.5 cm from the proximal end of femur, which contains the femoral head, neck, and greater trochanter) is determined. In order to determine the effects of polyphosphonates and statins on long bone metaphyses, histomorphometric analyses are performed on the proximal tibiae.

### Example 2: Reduction of Cholesterol levels of 0.2% Cholesterol-Fed New-Zealand White Rabbits

New Zealand White rabbits (female, aged 3-4 months, weighing less than 3 Kg), six in each group, are fed a control diet of 0.2% cholesterol (100 g rabbit chow daily containing 0.2 g cholesterol) or a diet of 0.2% cholesterol and a pharmaceutical composition containing a bone resorption inhibiting polyphosphonate or a diet of 0.2% cholesterol and a pharmaceutical composition containing a statin or a diet of 0.2% cholesterol and a pharmaceutical composition containing a bone resorption inhibiting polyphosphonate and a statin at a dose equivalent to the doses of the polyphosphonate and statin administered to the groups receiving diet containing only polyphosphonate and only statin. After 56 days, blood is collected from the rabbits and plasma and/or serum cholesterol levels are determined using the enzymatic method of Mao, et al., Clin.Chem. (1983) 29: 1890-1897.

## Claims

1. A pharmaceutical composition comprising:
(a) a polyphosphonate or an optical or geometric isomer thereof; or a pharmaceutically acceptable salt, N-oxide, ester, quaternary ammonium salt, or prodrug of either; and
(b) a statin or an optical or geometric isomer thereof; or a pharmaceutically acceptable salt, N-oxide, ester, quaternary ammonium salt or prodrug of either.

2. A pharmaceutical composition as claimed in claim 1 wherein the ployphosphonate is alendronic acid, alendronate, cimadronate, clodronic acid, clodronate, 1-hydroxy-3-(1-pyrrolidinyl)-propylidene-1,1-bisphosphonic acid, etidronic acid, ibandronate, neridronate, olpadronate, pamidronate, piridronate, risedronate, tiludronate or zolendronate.

3. A pharmaceutical composition as claimed in either of claims 1 and 2 wherein the statin is simvastatin, pravastatin, cerivastatin, mevastatin, fluindostatin, velostatin, fluvastatin, dalvastatin, dihydrocompactin, compactin, lovastatin, atorvastatin, bervastatin, NK-104 or ZD-4522.

4. A pharmaceutical composition as claimed in any preceding claim wherein the polyphosphonate is alendronate.

5. A pharmaceutical composition as claimed in any preceding claim wherein the statin is atorvastatin.

6. A pharmaceutical composition as claimed in claim 1 wherein the polyphosphonate is alendronate sodium or a hydrate thereof and said statin is atorvastatin hemicalcium salt or a hydrate thereof.

7. A pharmaceutical composition as claimed in any preceding claim further comprising an H₂ histamine receptor antagonist or a proton pump inhibitor or an optical or geometric isomer thereof; or a pharmaceutically acceptable salt, N-oxide, ester, quaternary ammonium salt, or prodrug thereof.

8. A pharmaceutical composition as claimed in any one of claims 1 to 7 for use as a medicament.

9. The use of a pharmaceutical composition, as claimed in any one of claims 1 to 7 in the manufacture of a medicament for promoting bone formation, preventing bone loss or treating atherosclerosis or any combination thereof.

10. A kit for use by a consumer to promote bone formation, prevent bone loss or treat atherosclerosis, or any combination thereof, said kit comprising
(a) a polyphosphonate or an optical or geometric isomer thereof; or a pharmaceutically acceptable salt, N-oxide, ester, quaternary ammonium salt, or prodrug of either; and
(b) a statin or an optical or geometric isomer thereof; or a pharmaceutically acceptable salt, N-oxide, ester, quaternary ammonium salt or prodrug of either; and, optionally,
(c) instructions describing a method of using the polyphosphonate and statin to promote bone formation, prevent bone loss or treat atherosclerosis, or any combination thereof.

11. A kit as claimed in claim 10 which further comprises an H₂ histamine receptor antagonist or a proton pump inhibitor or an optical or geometric isomer thereof; or a pharmaceutically acceptable salt, N-oxide, ester, quaternary ammonium salt, or prodrug thereof.

12. A kit as claimed in claim 10 wherein said polyphosphonate and/or statin is as defined in any one of claims 2 to 6.

13. A product containing
(a) a polyphosphonate or an optical or geometric isomer thereof; or a pharmaceutically acceptable salt, N-oxide, ester, quaternary ammonium salt, or prodrug of either; and
(b) a statin or an optical or geometric isomer thereof; or a pharmaceutically acceptable salt, N-oxide, ester, quaternary ammonium salt or prodrug of either; as a combined preparation for simultaneous, separate or sequential use to promote bone formation, prevent bone loss or treat atherosclerosis, or any combination thereof.

14. The use of
(a) a polyphosphonate or an optical or geometric isomer thereof; or a pharmaceutically acceptable salt, N-oxide, ester, quaternary ammonium salt, or prodrug of either; and
(b) a statin or an optical or geometric isomer thereof; or a pharmaceutically acceptable salt, N-oxide, ester, quaternary ammonium salt or prodrug of either;
for the manufacture of a medicament for simultaneous, separate or sequential use to promote bone formation, prevent bone loss or treat atherosclerosis, or any combination thereof.
